(19) Europäisches Patentamt
European Patent Office
Office européen des brevets

(11) Numéro de publication: **0 447 324 B1**

(12) # FASCICULE DE BREVET EUROPEEN

(45) Date de publication du fascicule du brevet: **20.12.95**

(51) Int. Cl.$^6$: **C07D 239/545**, C07D 473/06, A61K 31/52, A61K 31/505

(21) Numéro de dépôt: **91400699.4**

(22) Date de dépôt: **14.03.91**

(54) **Dérivés de pyrimidinedione-2,4 et médicaments les contenant**

(30) Priorité: **15.03.90 FR 9003342**

(43) Date de publication de la demande:
**18.09.91 Bulletin 91/38**

(45) Mention de la délivrance du brevet:
**20.12.95 Bulletin 95/51**

(84) Etats contractants désignés:
**AT BE CH DE DK ES FR GB GR IT LI LU NL SE**

(56) Documents cités:
**EP-A- 0 092 398**
**FR-A- 2 232 320**
**FR-A- 2 531 085**
**US-A- 2 781 344**

**CHEMICAL ABSTRACTS, vol. 112, no. 17, 23 avril 1990, page 688, résumé no. 157937q, Columbus, OHIO, USA; B.A. PRIIMENKO et al.: "Synthesis of 7-alkyl-8-thioxanthines" & UKR. KHIM. ZH. (RUSS. ED.) 1989, 55(6), 650-3**

(73) Titulaire: **SANOFI**
**32-34, rue Marbeuf**
**F-75008 Paris (FR)**

Titulaire: **SOCIETE NATIONALE ELF AOUITAI-NE**
**Tour Elf,**
**2, Place de la Coupole,**
**La Défense 6**
**F-92400 Courbevoie (FR)**

(72) Inventeur: **Fraisse, Laurent**
**14 bis rue Michel de Coulom**
**F-64110 Jurancon (FR)**
Inventeur: **Le Fur, Gérard**
**19 ter, rue des Carrières**
**F-95160 Montmorency (FR)**
Inventeur: **Verlhac, Jean-Baptiste**
**10, rue Blaise Pascal**
**F-33400 Talence (FR)**

(74) Mandataire: **Polus, Camille et al**
**c/o Cabinet Lavoix**
**2, Place d'Estienne d'Orves**
**F-75441 Paris Cedex 09 (FR)**

**EP 0 447 324 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

CHEMICAL ABSTRACTS, vol. 84, no. 15, 12 avril 1976, page 576, résumé no. 105545u, Columbus, Ohio, US; J. ZAJACZKOWSKA: "New drugs in the group of xanthine derivatives. XXXVI. 8-substituted derivatives of 1-hexyltheobromine", & ACTA PHARM. JUGOSL. 1975, 25(4), 237-40

RECUEIL DES TRAVAUX CHIMIOUES DES PAYS-BAS, vol. 83, no. 10, octobre 1964, pages 1215-1229, La Haye, NL; H. DOLMAN et al.: "Diuretics I. 8- sulfamoyltheophylline and 7-substituted derivatives"

Pro. Natl. Acad. Sci. USA 78(1981), 6858

## Description

La présente invention concerne des dérivés de pyrimidinedione-2,4 et les médicaments qui les contiennent.

Ces composés notamment présentent une activité anti-radicalaire et sont des inhibiteurs de la peroxydation des lipides des membranes biologiques.

On sait que l'acide urique et certains de ses dérivés N-méthylés piègent les radicaux libres in vitro et il a été envisagé dans Pro. Natl. Acad. Sci. USA 78 6858 (1981) que l'acide urique pouvait jouer, chez l'homme, un rôle protecteur contre la toxicité des radicaux oxygénés. Mais on a montré que l'acide urique n'est actif que sur certaines de ces espèces réactives puisqu'il n'inhibe pas la peroxydation des lipides membranaires, contrairement aux composés de la présente invention.

FR-A-2 232 320 décrit des amino-uraciles aux propriétés anticholestérolémiantes ne comportant pas de substituant alkyle à longue chaîne. Chem. Abs. Vol. 112 (17), (1990), 688 n° 157937 décrit l'utilisation de dérivés de l'acide urique, proches des composés de l'invention aux fins d'une étude des interactions hôte-récepteur.

EP-A-0 092 398 concerne des 8-phénylxanthines préparées à partir de 5,6-diaminouraciles proches des composés de l'invention mais ne comportant pas de substituant alkyle à longue chaîne.

FR-A-2 531 085 illustre de même la préparation de dérivés de la xanthine à partir de 5,6-diaminouraciles ne présentant pas non plus de groupes alkyles à longue chaîne.

Chem. Abs. 84 (1976) 576 n° 105545u décrit la synthèse de dérivés de la 1-hexylthéobromine substitués en position 8.

Recueil des travaux chimiques des Pays-Bas, vol. 83, (1964) 1215-1229 concerne la préparation de dérivés de la xanthine aux propriétés diurétiques.

US-A-2 781 344 propose des dérivés de 4,5-diaminouracile comme intermédiaires de synthèse de dérivés purines.

Les composés de l'invention répondent à la formule

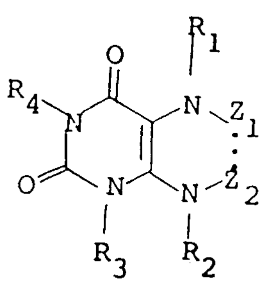

I

dans laquelle
$R_1$ représente H ou ($C_7$-$C_{20}$) alkyle,
$R_2$ représente H ou ($C_7$-$C_{20}$) alkyle,
$R_3$ représente H, $CH_3$ ou ($C_7$-$C_{20}$) alkyle,
$R_4$ représente H, $CH_3$ ou ($C_7$-$C_{20}$) alkyle,
$Z_1$ et $Z_2$ représentent chacun H ou représentent ensemble le groupe carbonyle ou thiocarbonyle,
étant entendu que l'un seul des substituants $R_1$ à $R_4$ représente un groupe ($C_7$-$C_{20}$)alkyle et que l'un des $R_3$ ou $R_4$ est différent de H lorsque $R_1$ représente n-dodécyle, ainsi que les sels d'addition de ces composés avec les acides minéraux et organiques pharmaceutiquement acceptables. Le disclaimer a été introduit afin d'écarter le composé de la formule I dans laquelle $R_1$ = n-$C_{12}H_{25}$, $R_2 = R_3 = R_4 = H$ et $Z_1 ... Z_2$ = $\rangle C=O$, décrit dans J. Amer. Chem. Soc. 109, (1987), 6549 - 6551. Dans cette publication on divulge une étude des interactions hôte-récepteur dans le cas de dérivés de l'acide urique.

Les composés de formule I peuvent être préparés de la manière suivante :

1°) on introduit un groupe amino $NR_1Z_1$ en position 5 d'un composé de formule

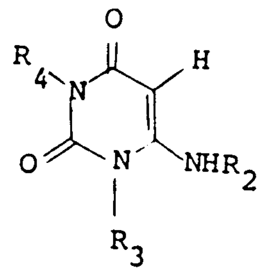

V

- soit par réduction d'un groupe NO ou $NO_2$ introduit en position 5 respectivement par nitrosation ou nitration de ce composé (V) pour obtenir les composés dans lesquels $Z_1 = R_1 = H$
- soit par action de $HNR_1Z_1$ sur le dérivé bromé en position 5, obtenu par bromation de ce composé (V) pour obtenir les composés dans lesquels $Z_1 = H$ et $R_1$ représente ($C_7$-$C_{20}$) alkyle
  et éventuellement, pour obtenir les composés dans lesquels $Z_1$ et $Z_2$ représentent ensemble le groupe carbonyle ou thiocarbonyle,

2°) on fait réagir respectivement l'urée ou la thiourée sur le composé de formule

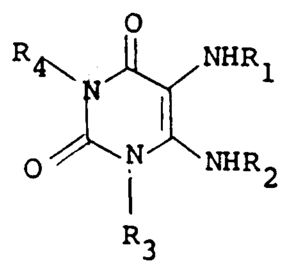

II

éventuellement par l'intermédiaire du carbamate en position 5 lorsque $R_1$ représente ($C_7$-$C_{20}$) alkyle et $R_2$ représente H.

Ce procédé est expliqué de manière plus détaillée ci-dessous.

Les composés de formule II

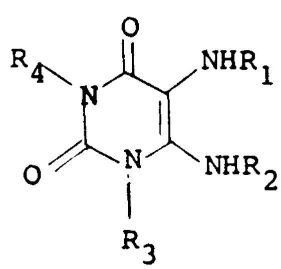

II

dans laquelle $R_1$ représente H et $R_2$, $R_3$ et $R_4$ sont comme dans la formule I peuvent être préparés à partir des dérivés de formule III, soit par l'intermédiaire du formamide (IV) selon le schéma réactionnel (a)

a)

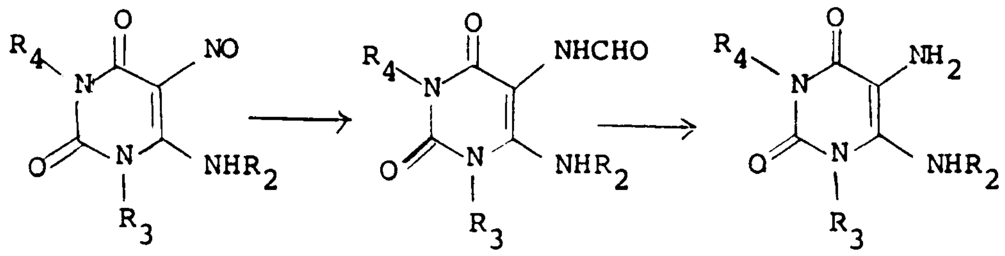

III IV

soit directement par hydrogénation catalytique ou par action d'un dithionite alcalin en milieu aqueux ou dans le formamide.

Le formamide (IV) est obtenu, par exemple, par action du zinc ou de dithionite alcalin et d'acide formique.

Certains dérivés de formule III sont connus; les autres pourront être préparés, de façon connue en soi, par action d'un nitrite d'alkyle, comme le nitrite d'isoamyle, ou d'un nitrite alcalin sur les pyrimidinediones de formule V

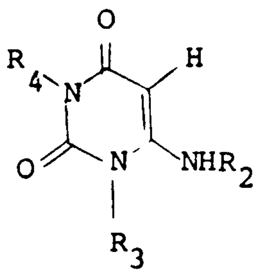

V

dans laquelle $R_2$, $R_3$ et $R_4$ sont comme dans la formule I.

Les composés de formule II dans lesquels $R_1$ représente H peuvent aussi être préparés à partir de chloro-6 pyrimidinedione-2,4 selon le schéma réactionnel (b)

b)

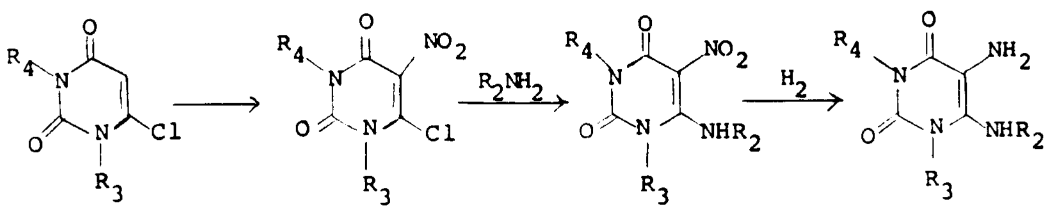


en appliquant des méthodes classiques, comme celle décrite dans Liebigs Ann. Chem. 677 p. 113 (1964).

Les composés de formule II dans lesquels $R_1$ est un groupe ($C_7$-$C_{20}$) alkyle sont préparés par action de l'amine $R_1NH_2$ sur le dérivé bromé de formule VI

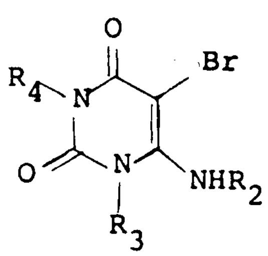

VI

dans laquelle $R_2$, $R_3$ et $R_4$ sont comme dans la formule I. Le dérivé (VI) est obtenu par action du brome dans l'acide acétique ou de la N-bromo succinimide dans un mélange d'acide et d'anhydride acétique sur

le composé (V), comme décrit dans Liebigs Ann. Chem. p. 1847-54 (1979); lorsque $R_3$ représente H il est préférable, avant d'effectuer la bromation, de protéger l'azote par exemple par un groupe benzyle, qui sera éliminé par hydrogénation catalytique après la réaction avec $R_1NH_2$.

Les composés de formule VII

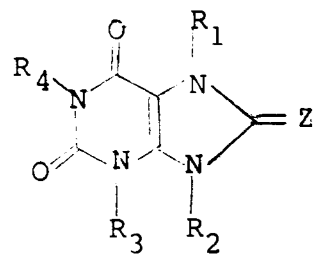

dans laquelle $R_1$ à $R_4$ sont comme dans la formule I et Z est O ou S, peuvent être préparés par action de l'urée ou de la thiourée sur les diaminopyrimidinediones de formule II en appliquant la méthode décrite dans Liebigs Ann. Chem. p. 2030 (1974) pour des produits dans lesquels $R_1$, $R_2$ = H et $R_3$, $R_4$ = H ou $CH_3$.

Lorsque $R_1$ représente un groupe ($C_7$-$C_{20}$) alkyle, on prépare de préférence le composé de formule VII en appliquant un procédé décrit dans Liebigs Ann. Chem. p. 1847-54 (1979) pour un composé II dans lequel $R_1$ = $CH_3$. Pour le composé dans lequel $R_2$ = $R_3$ = $R_4$ = H, il est nécessaire de bloquer l'azote portant $R_3$ et le schéma réactionnel est le suivant :

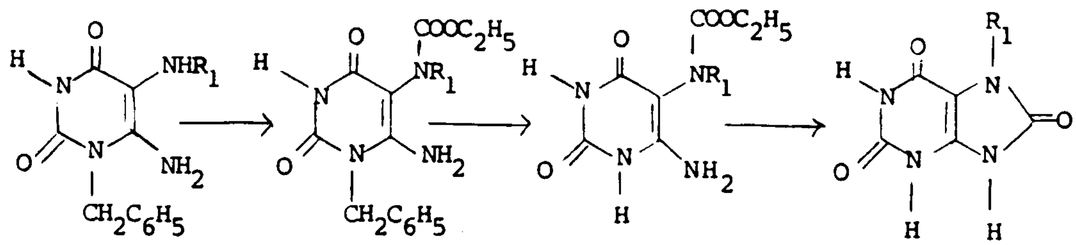

Le carbamate est préparé de façon classique par action d'un chloroformiate d'alkyle sur le composé (II) dans lequel $R_2$ = H,$R_3$ = $CH_2C_6H_5$ et $R_4$ = H, en présence d'une base, tandis que la cyclisation est effectuée par chauffage prolongé en milieu basique dans un solvant polaire, tel qu'un alcool, ou sans solvant en tube scellé en présence d'alcoolate de sodium, comme décrit dans Liebigs Ann. Chem. p. 1847 (1979).

Les amino-6 pyrimidinediones de départ (V), peuvent être préparées par des procédés dont les principes sont connus, qui ont été notamment décrits pour des composés dans lesquels $R_2$, = H et $R_3$ ou $R_4$, identiques ou différents, représentent H ou $CH_3$. Ainsi les composés de formule V

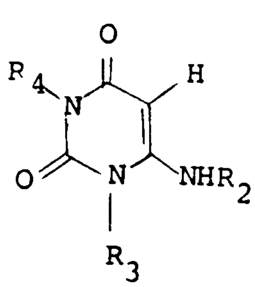

V

dans laquelle $R_3$ est différent de H et $R_2$ = $R_4$ = H, sont préparés par le procédé décrit dans J. Am. Chem. Soc. 63 2567 (1941), dans un alcool selon le schéma réactionnel (c) :

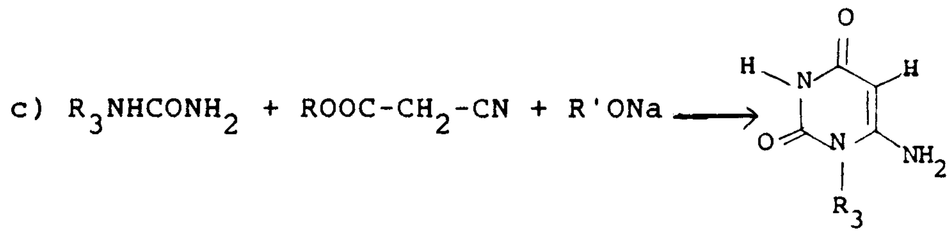


V

bis dans lequel R, R′ représentent un groupe alkyle en $C_1$ à $C_4$.

Pour obtenir les composés (V) dans lesquels $R_3$ et $R_4$ sont différents de H, on traite le composé de formule V bis, en milieu basique, par un composé $R_4X$ dans lequel X représente un atome d'halogène ou lorsque $R_4$ représente $CH_3$ de préférence par $(CH_3)_2SO_4$.

Les composés de formule V dans laquelle $R_3$ et $R_4$ représentent H sont préparés par action sur la chloro-6 pyrimidinedione, composé connu, de $R_2NH_2$ en milieu acide selon le schéma réactionnel (d) :

d)

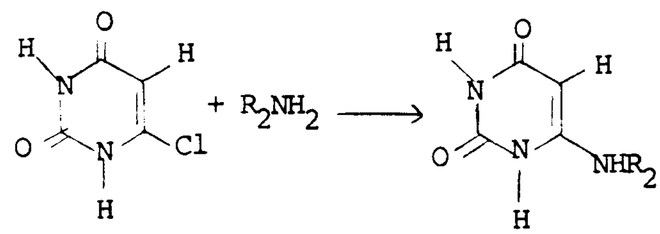


comme décrit, par exemple, dans J. Med. Chem. 20 p. 1186 (1977) ou par fusion du composé (V) dans lequel $R_2$ = $R_3$ = $R_4$ = H avec $R_2NH_2$ en présence de $R_2NH_2$, HCl.

Les composés de formule V dans laquelle $R_2$ et $R_3$ représentent H, sont préparés par le procédé décrit dans Liebigs Ann. Chem. 631 p. 168 (1960) selon le schéma réactionnel (e)

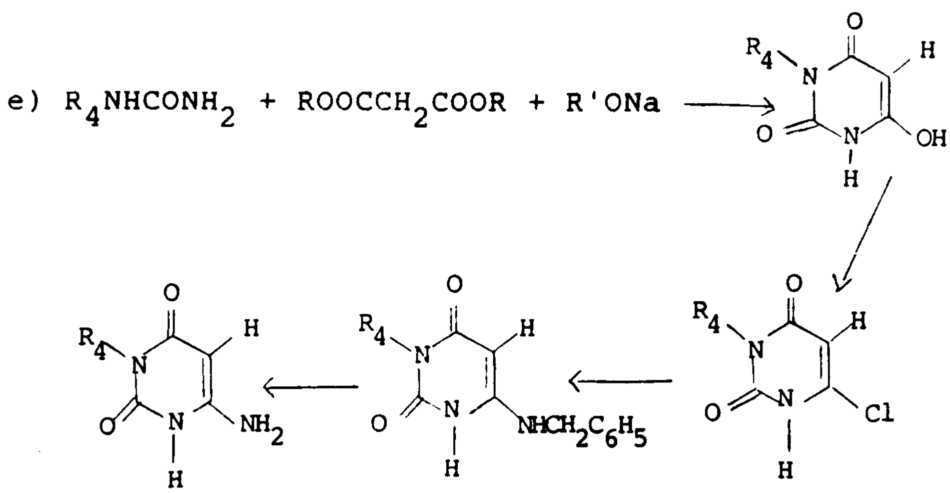


dans lequel R et R′ représentent chacun un groupe alkyle en $C_1$ à $C_4$, en appliquant le mode opératoire décrit dans Liebigs Ann. Chem. 612 p. 158 (1958).

Les sels des composés de formule I qui portent des fonctions amines sont préparés par action d'au moins un équivalent de l'acide sur l'amine en solution, ils sont isolés par précipitation ou par évaporation du solvant et purifiés par recristallisation s'il y a lieu.

Les compositions pharmaceutiques et les médicaments ayant pour principe actif au moins l'un des composés de formule I, dans laquelle

$R_1$ représente H ou ($C_7$-$C_{20}$) alkyle,
$R_2$ représente H ou ($C_7$-$C_{20}$) alkyle,
$R_3$ représente H, $CH_3$ ou ($C_7$-$C_{20}$) alkyle,
$R_4$ représente H, $CH_3$ ou ($C_7$-$C_{20}$) alkyle,

$Z_1$ et $Z_2$ représentent chacun H ou représentent ensemble le groupe carbonyle ou thiocarbonyle,
étant entendu que l'un seul des substituants $R_1$ à $R_4$ représente un groupe $(C_7\text{-}C_{20})$alkyle et que l'un des $R_3$ ou $R_4$ est différent de H lorsque $R_1$ représente n-dodécyle,
ou l'un des sels avec un acide pharmaceutiquement acceptable, sont un autre objet de l'invention; on préfère particulièrement les composés dans lesquels l'un des symboles $R_1$, $R_2$, $R_3$ ou $R_4$ représente un groupe alkyle en $C_{12}$ ou $C_{20}$.

La présence de radicaux libres en quantité excessive a été mise en évidence dans de nombreux états pathologiques et il est maintenant pratiquement démontré que les destructions tissulaires observées dans l'arthrite rhumatoïde, les maladies auto-immunes, les surcharges en fer, les suites d'ischémies cérébrales et cardiaques et certaines pathologies oculaires dépendent largement de la formation de radicaux libres. Aussi on peut envisager d'administrer à l'homme des composés qui inhibent la formation de ces espèces très réactives, à titre préventif ou curatif dans le traitement des maladies précédemment citées comme vraisemblablement dans les maladies de l'âge mûr pour lesquelles des radicaux libres ont été associés aux phénomènes dégénératifs observés.

On sait que parmi les radicaux libres, ceux dérivés de l'oxygène, et particulièrement le radical hydroxyle $OH^\bullet$, ont un rôle important; s'il est encore actuellement impossible de mesurer directement in vivo la concentration de ces radicaux, on sait l'estimer en étudiant la neutralisation de divers radicaux libres in vitro, et surtout la peroxydation des lipides membranaires, dont la conséquence est in vivo la formation d'aldéhydes cytotoxiques et la destruction des membranes cellulaires entraînant une augmentation de leur perméabilité.

L'action anti-radicalaire d'un produit XH est couramment déterminée, in vitro, par la mesure de la neutralisation du radical diphényl-1,1, picryl-2 hydrazyle ($DPPH^\bullet$), stable à 20 °C, par le produit XH dans la réaction $DPPH^\bullet + XH \rightarrow DPPH_2 + X^\bullet$

La disparition du radical $DPPH^\bullet$ est étudiée par spectrophotométrie à 517 nm, longueur d'onde à laquelle le radical absorbe fortement le rayonnement contrairement à la molécule d'hydrazine $DPPH_2$. Cette méthode est notamment décrite dans Free Radical in Biology & Medecine 3 p. 251-257 (1987) pour l'acide urique et ses dérivés N-méthylés.

Les composés de l'invention sont, dans cet essai, actifs à des doses comparables à celles de l'acide urique.

L'inhibition de la peroxydation lipidique due aux radicaux libres oxygénés, peroxydation particulièrement importante en présence de fer, a été déterminée, in vitro, par la mesure de la quantité d'aldéhyde malonique formée dans des suspensions de mitochondries, mises en présence d'un générateur d'ions superoxydes comme l'acide dihydroxyfumarique.

L'acide urique est inactif dans cet essai, alors que les composés selon l'invention ont une activité voisine de celle des amino-2 stéroïdes décrits dans Drugs of the future vol. 14 (2), 143-152, (1989) et notamment du 21-[4-(3,6-bis(diéthylamino)-2-pyridinyl)-1 piperazinyl]-16 α-méthylpregna-1,4,9(11)triene-3,20-dione (composé A).

Les compositions pharmaceutiques selon l'invention comprenant comme principe actif au moins l'un des composés de formule I ou l'un de ses sels avec un acide pharmaceutiquement acceptable seront administrés pour le traitement préventif ou curatif des phénomènes dégénératifs liés à la présence de radicaux libres, par voie orale,parentérale, transmuqueuse ou percutanée. Les doses unitaires et journalières dépendront du composé, de la nature et de la gravité de la maladie, du poids et de l'état du sujet ainsi que de la voie d'administration; en général, par voie orale la dose unitaire sera chez l'adulte de 1 mg à 500 mg, tandis que par voie intraveineuse, elle sera de 0,1 à 25 mg.

Les composés de formule I et leurs sels avec des acides pharmaceutiquement acceptable trouvent en outre une application dans des compositions cosmétiques pour s'opposer au vieillissement de la peau.

On décrit dans ce qui suit des exemples de composés de l'invention, leur procédé de préparation et les résultats obtenus lors de l'étude de leurs activités anti-radicalaire et anti-lipoperoxydante ainsi que celles du composé de référence A précédemment cité.

Les résultats des analyses centésimales des composés isolés sont compatibles avec leurs formules brutes; les points de fusion indiqués sont instantanés. Les spectres $RMN^1H$ ont été traçés en solution dans le diméthylsulfoxyde deutéré, avec comme étalon interne le tétraméthylsilane.

On décrit d'abord des exemples de préparation des amino-6 pyrimidines dione-2,4 de formule V.

A) Alkylamino-6 pyrimidinedione-2,4

i) formule V : $R_2 = C_{12}H_{25}$, $R_3 = H, R_4 = H$

On maintient à 160 °C durant 3 heures un mélange de 7,5 g d'amino-6 pyrimidinedione-2,4, de 12 g de

dodécylamine et 12 g de chlorhydrate de dodécylamine. Le milieu réactionnel est alors ramené à température ambiante avant l'introduction de 100 ml d'éthanol.
Le solide est isolé par filtration puis mis en suspension pendant quelques minutes dans 100 ml d'une solution aqueuse de NaOH 2N. Le solide restant est isolé et recristallisé dans l'acide acétique. On obtient ainsi 7 g du produit attendu fondant à plus de 250 °C.
RMN(300 MHz) $\delta$ : 0,85(t,3H); 1,25(m,18H); 1,50(m,2H); 2,98(q,2H); 4,37(s,1H); 5,98(t,1H); 9,78(s,1H); 10,07-(s,1H).

ii) On prépare de la même façon à partir d'hexadécylamine le dérivé de formule V dans laquelle $R_2 = C_{16}H_{33}$, $R_3 = H$, $R_4 = H$ qui fond à plus de 250 °C.

B) Alkyl-1 amino-6 pyrimidinedione-2,4

Formule V : $R_3 = C_{12}H_{25}$, $R_2 = H$, $R_4 = H$

On introduit dans une solution d'éthylate de sodium préparée avec 4 g de sodium dans 100 ml d'éthanol, 17,6 g de cyanoacétate d'éthyle et 35 g de dodécylurée. Le milieu est maintenu à sa température de reflux durant 7 heures, puis on introduit, après retour à température ambiante, une solution aqueuse concentrée de HCl jusqu'à pH acide et ensuite 200 ml d'eau. Le précipité formé est isolé par filtration puis recristallisé dans l'éthanol. On isole ainsi 29g du produit cherché qui fond à plus de 250 °C.
RMN(300 MHz) $\delta$ : 0,87(t,3H); 1,26(m,18H); 1,52(m,2H); 3,73(t,2H); 4,58(s,1H); 6,69(m,2H).

C) Alkyl-1 alkyl-3 amino-6 pyrimidinedione-2,4

Formule V : $R_3 = C_{12}H_{25}$, $R_2 = H$, $R_4 = CH_3$.

On met en suspension dans 10 ml d'une solution aqueuse de NaOH et 10 ml d'éthanol, 2,95 g de l'amino-6 dodécyl-1 pyrimidinedione-2,4 préparée en B et à 60 °C, on introduit 1,3 g de $(CH_3)_2SO_4$. Le chauffage est maintenu jusqu'à ce que le pH du milieu soit pratiquement neutre; on introduit alors une solution aqueuse de NaOH N jusqu'à pH basique et, après refroidissement, on extrait la solution par du chloroforme. La phase organique, lavée à l'eau et séchée est amenée à siccité. On obtient ainsi 2,35 g du produit attendu qui fond à 74 °C.

D) Alkyl-3 amino-6 pyrimidinedione-2,4

Formule V : $R_2 = H$, $R_3 = H$, $R_4 = C_{12}H_{25}$.

a) On introduit dans 150 ml d'une solution d'éthylate de sodium préparée avec 3,5 g de sodium, 35 g de dodécylurée et 24,3 g de malonate d'éthyle; le mélange est maintenu 8 heures à sa température de reflux, avant l'addition, à température ambiante d'une solution aqueuse d'acide chlorhydrique concentré jusqu'à pH acide. Le précipité est alors isolé par filtration, lavé à l'eau puis recristallisé dans l'éthanol. On obtient ainsi 30 g de dodécyl-1 pyrimidinetrione-2,4,6. F = 129-130 °C.

b) On met en suspension 30 g du produit obtenu selon a) dans 13,5 ml d'eau et on additionne goutte à goutte 102 ml de $POCl_3$. Le mélange est ensuite maintenu à sa température de reflux pendant une heure, puis l'excès de $POCl_3$ distillé sous pression réduite. Le résidu est versé sur de la glace pilée et le précipité formé est isolé. Après lavage à l'eau et recristallisation dans un mélange eau-méthanol, on obtient 20,5 g de chloro-6 dodécyl-3 pyrimidinedione-2,4 qui fond à 156-157 °C.

c) On maintient à 170 °C durant 1 heure un mélange de 30 g du dérivé chloré obtenu selon b) et 150g de benzylamine. Après avoir ramené le milieu à température ambiante, on y introduit 100 ml d'eau et isole le précipité apparu. Après lavage de celui-ci au méthanol et à l'éther éthylique, on récupère 25,13 g de benzylamino-6 dodécyl-3 pyrimidinedione-2,4 qui fond à 191 °C.

d) On met en suspension dans 400 ml d'éthanol absolu 25 g du composé obtenu selon c) et 4g de palladium sur charbon à 10%, dans un appareil à hydrogéner dans lequel on maintient pendant 6 heures une pression d'hydrogène de 3 MPa et une température de 80 °C. Le catalyseur est éliminé par filtration de la solution encore chaude avant d'évaporer le solvant sous pression réduite et le solide résiduel est lavé avec de l'éther éthylique. On isole ainsi 17,02 g d'amino-6 dodécyl-3 pyrimidinedione-2,4 qui fond à 242 °C.

Dans ce qui suit, on décrit la préparation de composés de formule III.

E) Amino-6 dodécyl-1 nitroso-5 pyrimidinedione-2,4 (Formule III : $R_2$ = H; $R_3$ = $C_{12}H_{25}$; $R_4$ = H).

On introduit goutte à goutte 5 ml de nitrite d'isoamyle dans 120 ml d'éthanol dans lesquels 5 g d'amino-6 dodécyl-1 pyrimidinedione-2,4 sont en suspension, puis 2 gouttes d'une solution aqueuse concentrée de HCl. Après 3 heures d'agitation à température ambiante, les cristaux violets formés sont isolés et recristallisés dans l'éthanol. On obtient ainsi 4,35 g du produit attendu qui fond à 206 °C.

Celui-ci peut aussi être représenté par la formule tautomère:

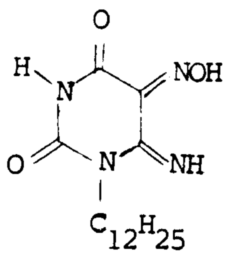

En appliquant le mode opératoire décrit en E, on a préparé les composés dont les caractéristiques figurent dans le tableau 1 de formule :

| | $R_2$ | $R_3$ | $R_4$ | F °C |
|---|---|---|---|---|
| F | H | $C_{12}H_{25}$ | $CH_3$ | 158 |
| G | $C_{16}H_{33}$ | H | H | 168 |
| H | $C_{12}H_{25}$ | H | H | 173 |
| I | H | H | $C_{12}H_{25}$ | 230 |

EXEMPLE 1

Diamino-5,6 dodécyl-1 méthyl-3 pyrimidinedione-2,4 (formule I : $Z_1$ = $Z_2$ = H; $R_1$ = $R_2$ = H; $R_3$ = $C_{12}H_{25}$; $R_4$ = $CH_3$).

a) Dans une solution portée à sa température de reflux, de 4,35 g du composé de l'exemple C dans 100 ml d'acide formique, on ajoute par petites portions 4 g de zinc en poudre; après 1 heure de reflux, on filtre à chaud et évapore les solvants du filtrat sous pression réduite. On verse sur le résidu 30 ml de $CH_3OH$ et isole le précipité.

On obtient ainsi 3,85 g d'amino-6 dodécyl-1 formylamino-5 méthyl-3 pyrimidinedione-2,4 qui fond à 215 °C.

b) On dissout 3,63 g du composé obtenu en a) dans 100 ml de $CH_3OH$, dans lequel on introduit de l'acide chlorhydrique gazeux par un léger bullage maintenu durant 15 minutes. La solution est ensuite maintenue 3 heures à sa température de reflux, puis refroidie à 0 °C. On filtre alors le précipité pour isoler 3,21 g de monochlorhydrate du produit final qui fond à 195 °C.

On peut préparer la diamine en traitant une suspension de son chlorhydrate dans $CH_3OH$ par une solution aqueuse concentrée de $NH_4OH$.

EXEMPLES 2 à 7

Les composés de formule I dans laquelle $Z_1 = Z_2 = H$ dont les caractéristiques figurent dans le tableau 2, sont préparés en appliquant le procédé de l'exemple 1.

| Exemple | $R_1$ | $R_2$ | $R_3$ | $R_4$ | F °C (sel) |
|---|---|---|---|---|---|
| 2 | H | H | $C_{12}H_{25}$ | H | 237 |
| 3 | H | H | H | $C_{12}H_{25}$ | >250 (HCl) |
| 4 | H | $C_{16}H_{33}$ | H | H | 242 (HCl) |
| 5 | H | $C_{12}H_{25}$ | H | H | >200 (HCl) |
| 6 | H | H | $C_{10}H_{21}$ | H | >250 (HCl) |
| 7 | H | H | $C_8H_{17}$ | H | >200 (HCl) |

RMN EX 6 : (500 MHz) δ : 0,84(t,3H); 1,25(m,14H); 1,51(m,2H); 3,81(t,2H); 7,72(s,2H); 9,75(s,3H); 11,10-(s,1H).

EX 7 : (250 MHz) δ : 0,85(t,3H); 1,25(m,10H); 1,49(m,2H); 3,80(t,2H); 7,81(s,2H); 9,46(s,3H); 11,13 (s,1H).

EXEMPLES 8 et 9

Dodécyl-3 méthyl-1 [1H,3H,9H] purinetrione-2,6,8. (formule I : $Z_1$ et $Z_2$ = CO; $R_1$ = $R_2$ = H; $R_3$ = $C_{12}H_{25}$; $R_4$ = $CH_3$).

On maintient à 180 °C durant 1 heure un mélange de 800 mg du composé obtenu à l'exemple 1 avec 1 g d'urée. A température ambiante, on verse dans le milieu 20 ml d'eau; le solide est isolé, lavé avec de l'éthanol et recristallisé dans l'acide acétique. On isole ainsi 680 mg du produit recherché fondant à 266 °C.
RMN (250MHz) -δ : 0,86(t,3H); 1,24(m,18H); 1,56(m,2H); 3,19(s,3H); 3,77(t,2H); 10,79(s,1H); 11,89(s,1H).

En appliquant le même mode opératoire avec la thiourée, on obtient l'octyl-3[1H,3H,9H]purinedione-2,6-thione-8 : F >250 °C.
RMN (90MHz) δ : 0,85(t,3H); 1,24(m,12H); 3,87(t,2H); 11,24(s,1H);12,98(s,1H); 13,47(s,1H).

EXEMPLES 10 à 15

Les composés de formule I dans laquelle $Z_1$, $Z_2$ représentent C=O dont les caractéristiques figurent dans le tableau 3, ont été préparés en appliquant le procédé mis en oeuvre à l'exemple 8.

| Exemple | $R_1$ | $R_2$ | $R_3$ | $R_4$ | F °C |
|---|---|---|---|---|---|
| 10 | H | H | H | $C_{12}H_{25}$ | >250 |
| 11 | H | $C_{12}H_{25}$ | H | H | >250 |
| 12 | H | $C_{16}H_{33}$ | H | H | >250 |
| 13 | H | H | $C_{12}H_{25}$ | H | >250 |
| 14 | H | H | $C_8H_{17}$ | H | >250 |
| 15 | H | H | $C_{10}H_{21}$ | H | >250 |

dont les RMN figurent dans le tableau 4.

TABLEAU 4

| Exemple | fréquence | δ |
|---|---|---|
| 10 | 300 MHz | 0,86(t,3H) ; 1,25(m,18H) ; 1,52(m,2H) ; 3,70(t,2H) ; 10,77(s,1H) ; 11,03(s,1H) ; 11,83(s,1H). |
| 11 | 300 MHz | 0,85(t,3H) ; 1,25(m,18H) ; 1,51(m,2H) ; 3,63(t,2H) ; 10,82(s,1H) ; 10,86(s,1H) ; 12,00(s,1H). |
| 12 | 250 MHz | 0,84(t,3H) ; 1,22(m,26H) ; 1,51(m,2H) ; 3,62(t,2H) ; 10,78(s,1H) ; 10,81(s,1H) ; 11,98(s,1H). |
| 13 | 250 MHz | 0,85(t,3H) ; 1,22(m,18H) ; 1,48(m,2H) ; 3,73(t,2H) ; 10,54(s,1H) ; 11,35(s,1H) ; 12,03(s,1H). |
| 14 | 250 MHz | 0,85(t,3H) ; 1,25(m,10H) ; 1,53(m,2H) ; 3,71(t,2H) ; 10,76(s,1H) ; 11,01(s,1H) ; 11,85(s,1H). |
| 15 | 500 MHz | 0,84(t,3H) ; 1,25(m,14H) ; 1,51(m,2H) ; 3,81(t,2H) ; 10,71(s,1H) ; 10,98(s,1H) ; 11,82(s,1H). |

EXEMPLE 16

Amino-5 octadécylamino-6 méthyl-3 pyrimidinedione-2,4 (formule I : $Z_1$ et $Z_2$ = H; $R_1$ = H; $R_2$ = $C_{18}H_{37}$; $R_3$ = H; $R_4$ = $CH_3$)

a) On introduit, par fractions, 2,05 g de chloro-6 méthyl-3 nitro-5 pyrimidinedione-2,4 résultant de la nitration de la chloro-6 méthyl-3 pyrimidinedione-2,4 selon un procédé décrit dans Liebigs Ann. Chem. 677 113 (1964), dans une solution de 3 g d'octadécylamine et 1 g de triéthylamine dans 20 ml d'éthanol. La solution est maintenue 20 minutes à sa température de reflux, puis neutralisée par addition d'acide acétique. Le précipité formé est lavé à l'eau et recristallisé dans l'éthanol.

On isole ainsi 2,11 g d'octadécylamino-6 méthyl-3 nitro-5 pyrimidinedione-2,4 qui fond à 130°C.

b) Le produit ainsi obtenu est mis en solution dans 50 ml d'acide formique et on introduit dans le milieu par petites portions 2 g de zinc en poudre; après 1 heure de reflux on filtre à chaud et évapore les solvants du filtrat sous pression réduite. Le résidu est cristallisé avec 15 ml de méthanol. On obtient ainsi 1,8 g de formamido-5 octadécylamino-6 méthyl-3 pyrimidinedione-2,4 qui fond à 196°C.

c) Le formamide obtenu ci-dessus est traité par HCl dans le méthanol comme à l'exemple 1-b.

On obtient ainsi le chlorhydrate du produit recherché qui fond à 187°C.

RMN(250MHz)δ: 0,89(t,3H); 1,27(m,30H); 1,55(m,2H); 3,15(s,3H); 3,32(m,2H); 7,86(s,1H); 10,40(s,3H).

L'activité antiradicalaire de ces composés a été déterminée comme suit : on introduit, dans 970 µl d'un mélange de méthanol et de solution aqueuse tamponnée par du TRIS (trométhamine), HCl (10 mM-pH 7,4) à raison de 1 volume de méthanol pour 2 de tampon, 20 µl d'une solution de DPPH• 1mM et 10 µl d'une solution du produit à tester dans le diméthylsulfoxyde. Le mélange est maintenu 20 minutes à 20°C avant de déterminer la densité optique à 517 nm par rapport à un mélange témoin pour lequel le produit à tester n'a pas été introduit dans le diméthylsulfoxyde.

Le pourcentage P de piégeage du radical libre est défini comme un rapport des densités optiques D

$$P = \frac{D(\text{produit}) - D(\text{témoin})}{D(\text{témoin})} \times 100$$

La concentration efficace 50 ($CE_{50}$), celle pour laquelle P = 50, est calculée sur la base des résultats des mesures faites avec 4 concentrations différentes dans une gamme de 10 autour de la $CE_{50}$.

Dans le tableau 5, figurent les $CE_{50}$ pour des composés selon l'invention, ainsi que pour l'acide urique et ses dérivés N-méthylés et pour le produit A.

TABLEAU 5

| PRODUIT | | $CE_{50}$ (µM) | PRODUIT | $CE_{50}$ (µM) |
|---|---|---|---|---|
| acide urique | | 4,5 | exemple 10 | 5,2 |
| acide urique | $CH_3$-1 | 4,5 | 11 | 6,2 |
| | $CH_3$-3 | 3,2 | 12 | 4,5 |
| | $CH_3$-7 | 20 | 13 | 3,2 |
| | $CH_3$-9 | 5,2 | | |
| A | | 4 | 14 | 4 |
| exemple | 1 | 5 | 15 | 3,4 |
| | 2 | 7 | | |
| | 3 | 5,5 | | |
| | 4 | 6,4 | 16 | 6,2 |
| | 5 | 6,4 | | |
| | 6 | 5,6 | | |
| | 7 | 6,5 | | |
| | 8 | 2,7 | | |
| | 9 | 6,5 | | |

L'activité inhibitrice de la peroxydation des lipides membranaires des composés a été déterminée comme suit :

a) préparation des mitochondries

On prélève un coeur de boeuf immédiatement après l'abattage de l'animal et le refroidit à 4°C, température à laquelle toutes les opérations suivantes seront effectuées. Le coeur est lavé avec une solution aqueuse tampon à pH 7,4 (TRIS,HCl 10 mM, EDTA 2 mM, KCl 20 mM, saccharose 250 mM) puis les tissus conjonctifs et les graisses sont éliminés. Le muscle est alors haché et le hachis est mis en suspension dans une solution aqueuse tampon à pH 7,4 (TRIS,HCl 10 mM, saccharose 250 mM, EDTA 1 mM) à raison de 700 g de muscle pour 3 litres. Le pH de la suspension est réajusté à 7,4, régulièrement, avant d'isoler le hachis par filtration sur une gaze. Il est ensuite remis en suspension dans 3 litres du second tampon et la suspension est broyée avant d'être centrifugée pendant 20 minutes à 1000 g, puis le surnageant est centrifugé 15 minutes à 17000 g. Le culot de centrifugation est lavé plusieurs fois par une solution aqueuse tampon de pH 7,4 (TRIS,HCl 15 mM, saccharose 250 mM) avec séparation par centrifugation de la solution de lavage et on prépare finalement une suspension homogène de mitochondries dans ce même tampon à 50 mg de protéines/ml qui sera conservée congelée en gouttelettes, dans l'azote liquide. Après décongélation les contrôles respiratoires réalisés en mesurant les vitesses de consommation d'oxygène en présence ou non d'ADP pour les substrats succinate et glutamate/malate, sont 80% de ceux obtenus avant la congélation, plusieurs mois après la préparation des mitochondries.

b) Lipoperoxydation

On disperse à 4°C une fraction de mitochondries décongelées dans une solution aqueuse tamponnée de pH 7,4 (TRIS,HCl 30 mM contenant KCl 140 mM), pour obtenir une concentration en protéines de 0,37 mg/ml; dans 960 µl de cette suspension, on introduit 10 µl d'une solution d'acide dihydroxyfumarique 100 mM dont le pH a été ajusté à pH 7,42 µl d'une solution du produit à tester en solution dans le diméthylsulfoxyde (DMSO) et 30 µl d'une solution de $FeSO_4,(NH_4)_2SO_4$ 1mM, conservée à l'abri de l'oxygène et de la lumière. L'échantillon témoin contient le DMSO sans le produit à tester tandis que le blanc ne contient ni acide dihydroxyfumarique, ni fer.

Après un premier prélèvement de 100 µl, les échantillons sont maintenus à 37°C sous légère agitation et des prélèvements de 100 µl y sont effectués après 20 et 40 minutes.

Chaque prélèvement est introduit immédiatement dans 1,5 ml d'une solution d'acide trichloroacétique 13,5% (p/v) et d'acide thiobarbiturique 0,33% (p/v) dans une solution aqueuse d'acide chlorhydrique 0,85 N et les récipients sont fermés puis portés à 100°C pendant 15 minutes, avant d'être refroidis jusqu'à température ambiante en 5 minutes. On ajoute alors 1 ml d'une solution aqueuse d'acide trichloroacétique à 70% (p/v) avant d'effectuer la mesure spectrofluorimétrique, avec une longueur d'onde d'excitation de 515 nm et d'émission de 553 nm.

Une gamme étalon de malonaldéhyde bis-(diéthylacétal) dilué dans une solution aqueuse de HCl 0,1 N permet de calculer à quelle concentration d'aldéhyde correspond chaque intensité de la fluorescence i.

Dans les conditions expérimentales, on a déterminé

$$C\ (\text{nmole}) = \frac{i - 1{,}89}{35{,}5}$$

et par conséquent, le pourcentage d'inhibition de la peroxydation I peut être calculé par la formule

$$I = \frac{(i - 1{,}89)\text{témoin} - (i - 1{,}89)\text{essai}}{(i - 1{,}89)\text{témoin} - (i - 1{,}89)\text{blanc}} \times 100$$

La concentration inhibant 50% du signal $CI_{50}$, c'est-à-dire celle pour laquelle I = 50, est calculée d'après les mesures effectuées avec 4 concentrations différentes du produit à tester dans une gamme de 10 autour de la $CI_{50}$.

Les résultats obtenus sont indiqués dans le tableau 6. L'acide urique n'inhibe pas la formation de malonaldéhyde à une concentration inférieure à 100 µM; ses dérivés N-méthylés sont peu actifs, pour le plus actif I = 35 à la concentration de 100 µM.

TABLEAU 6

| exemple | $CI_{50}$ (µM) | exemple | $CI_{50}$ (µM) |
|---|---|---|---|
| A | 0,7 | 10 | 1,25 |
| 1 | 0,24 | 11 | 0,3 |
| 2 | 0,27 | 12 | 0,9 |
| 3 | 0,26 | 13 | 1,6 |
| 4 | 0,9 | | |
| 5 | 0,3 | 14 | 28 |
| | | 15 | 3,7 |
| 6 | 0,28 | | |
| 7 | 0,62 | | |
| 8 | 0,85 | 16 | 20 |
| 9 | 25 | | |

**Revendications**

**Revendications pour les Etats contractants suivants : AT, BE, CH, DE, DK, FR, GB, GR, IT, LI, LU, NL, SE**

**1.** Composé de formule

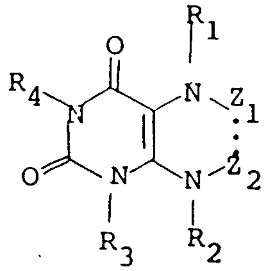

I

dans laquelle
$R_1$ représente H ou ($C_7$-$C_{20}$) alkyle,
$R_2$ représente H ou ($C_7$-$C_{20}$) alkyle,
$R_3$ représente H, $CH_3$ ou ($C_7$-$C_{20}$) alkyle,
$R_4$ représente H, $CH_3$ ou ($C_7$-$C_{20}$) alkyle,
$Z_1$ et $Z_2$ représentent chacun H ou représentent ensemble le groupe carbonyle ou thiocarbonyle,
étant entendu que l'un seul des substituants $R_1$ à $R_4$ représente un groupe ($C_7$-$C_{20}$)alkyle et que l'un des $R_3$ ou $R_4$ est différent de H lorsque $R_1$ représente n-dodécyle, et ses sels d'addition avec un acide pharmaceutiquement acceptable.

**2.** Composé selon la revendication 1, caractérisé en ce que l'un des $R_1$, $R_2$, $R_3$ ou $R_4$ représente un groupe alkyle en $C_{12}$ à $C_{20}$.

**3.** Composé selon l'une des revendications 1 ou 2, caractérisé en ce que $Z_1$ et $Z_2$ représentent chacun H.

**4.** Composé selon l'une des revendications 1 ou 2, caractérisé en ce que $Z_1$ et $Z_2$ représentent ensemble un groupe carbonyle ou thiocarbonyle.

**5.** Composé selon l'une des revendications 1 ou 2, caractérisé en ce que $Z_1$ et $Z_2$ représentent ensemble le groupe carbonyle.

**6.** Médicament contenant comme principe actif un composé selon la revendication 1.

**7.** Médicament selon la revendication 6, caractérisé en ce que $R_1$, $R_2$, $R_3$ ou $R_4$ représente un groupe alkyle en $C_{12}$ à $C_{20}$.

**8.** Médicament selon l'une des revendications 6 ou 7, caractérisé en ce que $Z_1$ et $Z_2$ représentent chacun H.

**9.** Médicament selon l'une des revendications 6 ou 7, caractérisé en ce que $Z_1$ et $Z_2$ pris ensemble représentent un groupe carbonyle ou thiocarbonyle.

**10.** Procédé de préparation des composés selon l'une des revendications 1 à 5 caractérisé en ce que
1°) on introduit un groupe amino $NR_1Z_1$ en position 5 d'un composé de formule

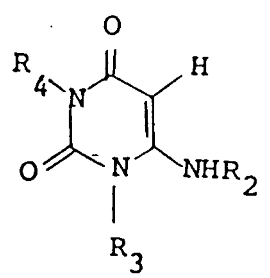

V

- soit par réduction d'un groupe NO ou $NO_2$ introduit en position 5 respectivement par nitrosation ou nitration de ce composé (V) pour obtenir les composés dans lesquels $Z_1$ = $R_1$ = H
- soit par action de $HNR_1Z_1$ sur le dérivé bromé en position 5, obtenu par bromation de ce composé (V) pour obtenir les composés dans lesquels $Z_1$ = H et $R_1$ représente ($C_7$-$C_{20}$) alkyle
  et éventuellement, pour obtenir les composés dans lesquels $Z_1$ et $Z_2$ représentent ensemble le groupe carbonyle ou thiocarbonyle,

2°) on fait réagir respectivement l'urée ou la thiourée sur le composé de formule

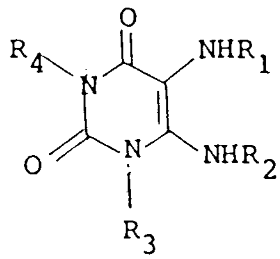

II

éventuellement par l'intermédiaire du carbamate en position 5 lorsque $R_1$ représente ($C_7$-$C_{20}$) alkyle et $R_2$ représente H.

**Revendications pour l'Etat contractant suivant : ES**

**1.** Procédé de préparation d'un composé de formule

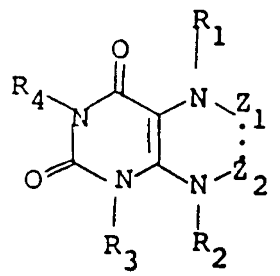

I

dans laquelle $R_1$
représente H ou ($C_7$-$C_{20}$) alkyle
$R_2$ représente H ou ($C_7$-$C_{20}$) alkyle
$R_3$ représente H, $CH_3$ ou ($C_7$-$C_{20}$) alkyle
$R_4$ représente H, $CH_3$ ou ($C_7$-$C_{20}$) alkyle et $Z_1$ et $Z_2$ représentent chacun H ou représentent ensemble le groupe carbonyle ou thiocarbonyle, étant entendu que l'un seul des substituants $R_1$ à $R_4$ représente un groupe ($C_7$-$C_{20}$) alkyle et que l'un des $R_3$ ou $R_4$ est different de H lorsque $R_1$ représente le groupe n-dodécyle,
et ses sels d'addition avec un acide pharmaceutiquement acceptable, caractérisé en ce que
1°) on introduit un groupe amino $NR_1Z_1$ en position 5 d'un composé de formule

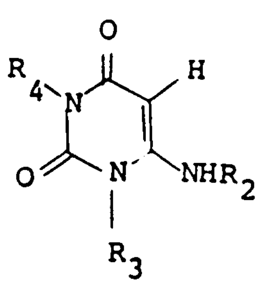

V

- soit par réduction d'un groupe NO ou $NO_2$ introduit en position 5 respectivement par nitrosation ou nitration de ce composé (V) pour obtenir les composés dans lesquels $Z_1$ = $R_1$ = H
- soit par action de $HNR_1Z_1$ sur le dérivé bromé en position 5, obtenu par bromation de ce composé (V) pour obtenir les composés dans lesquels $Z_1$ = H et $R_1$ représente ($C_7$-$C_{20}$) alkyle
  et éventuellement, pour obtenir les composés dans lesquels $Z_1$ et $Z_2$ représentent ensemble

le groupe carbonyle ou thiocarbonyle,

2°) on fait réagir respectivement l'urée ou la thiourée sur le composé de formule

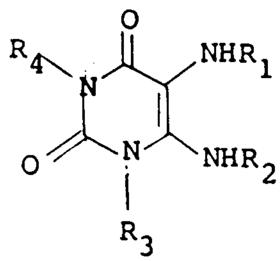

II

éventuellement par l'intermédiaire du carbamate en position 5 lorsque $R_1$ représente ($C_7$-$C_{20}$) alkyle et $R_2$ représente H.

**2.** Procédé selon la revendication 1, caractérisé en ce qu'on salifie en outre les composés de formule I dans lesquels $Z_1$ et $Z_2$ représentent H, par action d'un acide pharmaceutiquement acceptable.

**3.** Procédé selon l'une des revendications 1 ou 2, caractérisé en ce que l'un des $R_1$, $R_2$, $R_3$ ou $R_4$ représente un groupe alkyle en $C_{12}$ à $C_{20}$.

**4.** Procédé selon l'une des revendications 1 à 3, pour préparer un composé de formule I dans laquelle $Z_1$, $Z_2$ et $R_1$ représentent H, caractérisé en ce qu'on fait réagir un réactif de nitrosation sur le composé de formule V et que l'on réduit par hydrogénation catalytique le composé obtenu.

**5.** Procédé selon l'une des revendications 1 à 3, pour préparer un composé de formule I dans laquelle $Z_1$, $Z_2$ et $R_1$ représentent H, caractérisé en ce qu'on fait réagir un réactif de nitrosation sur le composé de formule V et que l'on réduit le dérivé nitroso obtenu par l'intermédiaire d'un formamide.

**6.** Procédé selon l'une des revendications 1 à 3, pour préparer un composé de formule I dans laquelle $Z_1$, $Z_2$ et $R_1$ représentent H, caractérisé en ce qu'on effectue une nitration du composé de formule V suivie d'une réduction du composé obtenu.

**7.** Procédé selon l'une des revendications 1 à 3, pour préparer un composé de formule I dans laquelle $Z_1$ et $Z_2$ représentent H et $R_1$ un groupe ($C_7$-$C_{20}$) alkyle, caractérisé en ce qu'on effectue une bromation du composé de formule V et que l'on fait réagir une amine de formule $H_2NR_1$, sur le composé obtenu.

**8.** Procédé selon l'une des revendications 3 à 7, pour préparer un composé de formule I dans laquelle $Z_1$ et $Z_2$ représentent ensemble un groupe carbonyle ou thiocarbonyle, caractérisé en ce qu'on fait réagir sur le composé de formule II respectivement l'urée ou la thiourée.

**9.** Procédé selon l'une des revendications 1, 3 et 7, pour préparer un composé de formule I dans laquelle $Z_1$ et $Z_2$ représentent ensemble un groupe carbonyle ou thiocarbonyle et $R_2$, $R_3$ et $R_4$ représentent H, caractérisé en ce qu'on fait réagir respectivement l'urée ou la thiourée sur le composé de formule

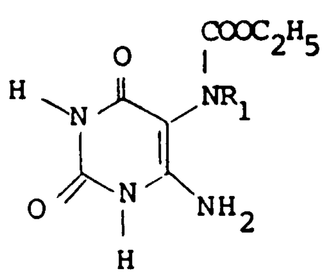

dans laquelle $R_1$ représente un groupe ($C_7$-$C_{20}$) alkyle, obtenu par débenzylation de

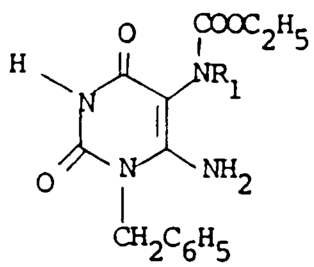

## Claims

**Claims for the following Contracting States : AT, BE, CH, DE, DK, FR, GB, GR, IT, LI, LU, NL, SE**

**1.** Compound of formula

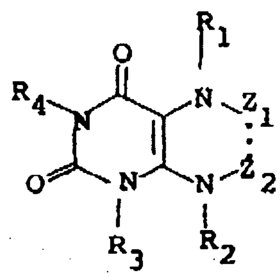

I

wherein
$R_1$ denotes H or $(C_{7-20})$alkyl,
$R_2$ denotes H or $(C_{7-20})$alkyl,
$R_3$ denotes H, $CH_3$ or $(C_{7-20})$alkyl,
$R_4$ denotes H, $CH_3$ or $(C_{7-20})$alkyl,
$Z_1$ and $Z_2$ each denote H or together represent the carbonyl or thiocarbonyl group,
on the understanding that only one of the substituents $R_1$ to $R_4$ denotes a $(C_{7-20})$alkyl group and either $R_3$ or $R_4$ is other than H when $R_1$ denotes n-dodecyl,
and the addition salts thereof with a pharmaceutically acceptable acid.

**2.** Compound according to claim 1, characterised in that one of $R_1$, $R_2$, $R_3$ or $R_4$ denotes a $C_{12-20}$-alkyl group.

**3.** Compound according to one of claims 1 and 2, characterised in that $Z_1$ and $Z_2$ each denote H.

**4.** Compound according to one of claims 1 and 2, characterised in that $Z_1$ and $Z_2$ together denote a carbonyl or thiocarbonyl group.

**5.** Compound according to one of claims 1 and 2, characterised in that $Z_1$ and $Z_2$ together denote the carbonyl group.

**6.** Medicament containing as active principle a compound according to claim 1.

**7.** Medicament according to claim 6, characterised in that $R_1$, $R_2$, $R_3$ or $R_4$ denotes a $C_{12-20}$-alkyl group.

**8.** Medicament according to one of claims 6 and 7, characterised in that $Z_1$ and $Z_2$ each denote H.

**9.** Medicament according to one of claims 6 and 7, characterised in that $Z_1$ and $Z_2$ taken together denote a carbonyl or thiocarbonyl group.

**10.** Process for preparing compounds according to one of claims 1 to 5, characterised in that

1°) an $NR_1Z_1$ amino group is introduced into position 5 of a compound of formula

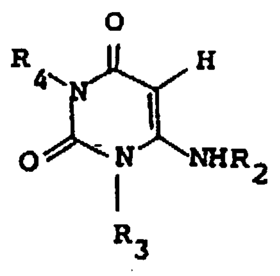

V

- either by reduction of an NO or $NO_2$ group introduced into the 5-position, respectively, by nitrosation or nitration of this compound (V) in order to obtain compounds wherein $Z_1 = R_1 = H$
- or by reacting $HNR_1Z_1$ with the derivative brominated in position 5, obtained by bromination of this compound (V), in order to obtain compounds wherein $Z_1$ = H and $R_1$ denotes $(C_{7-20})$alkyl and optionally, in order to obtain compounds wherein $Z_1$ and $Z_2$ together represent the carbonyl or thiocarbonyl group,

2°) urea or thiourea, respectively, is reacted with the compound of formula

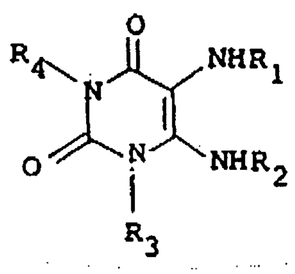

II

optionally via the carbamate in position 5 when $R_1$ denotes $(C_{7-20})$alkyl and $R_2$ denotes H.

**Claims for the following Contracting State : ES**

**1.** Process for preparing a compound of the formula

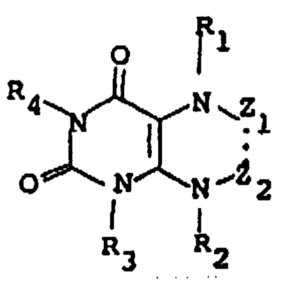

I

wherein
$R_1$ denotes H or $(C_{7-20})$alkyl,
$R_2$ denotes H or $(C_{7-20})$alkyl,
$R_3$ denotes H, $CH_3$ or $(C_{7-20})$alkyl,
$R_4$ denotes H, $CH_3$ or $(C_{7-20})$alkyl,
$Z_1$ and $Z_2$ each denote H or together represent the carbonyl or thiocarbonyl group,
on the understanding that only one of the substituents $R_1$ to $R_4$ denotes a $(C_{7-20})$alkyl group and either $R_3$ or $R_4$ is other than H when $R_1$ denotes n-dodecyl,
and the addition salts thereof with a pharmaceutically acceptable acid, characterised in that

1°) an $NR_1Z_1$ amino group is introduced into position 5 of a compound of formula

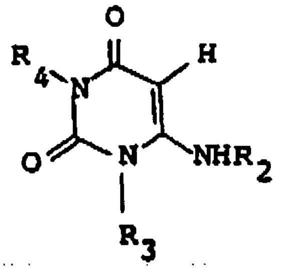

V

- either by reduction of an NO or $NO_2$ group introduced into the 5-position, respectively, by nitrosation or nitration of this compound (V) in order to obtain compounds wherein $Z_1 = R_1 = H$
- or by reacting $HNR_1Z_1$ with the derivative brominated in position 5, obtained by bromination of this compound (V), in order to obtain compounds wherein $Z_1 = H$ and $R_1$ denotes $(C_{7-20})$alkyl and optionally, in order to obtain compounds wherein $Z_1$ and $Z_2$ together represent the carbonyl or thiocarbonyl group,

2°) urea or thiourea, respectively, is reacted with the compound of formula

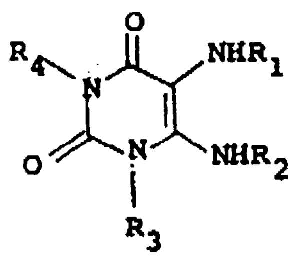

II

optionally via the carbamate in position 5 when $R_1$ denotes $(C_{7-20})$alkyl and $R_2$ denotes H.

2. Process according to claim 1, characterised in that in addition the compounds of formula I wherein $Z_1$ and $Z_2$ denote H are converted into salts by the action of a pharmaceutically acceptable acid.

3. Process according to one of claims 1 and 2, characterised in that one of $R_1$, $R_2$, $R_3$ or $R_4$ denotes a $C_{12-20}$ group.

4. Process according to one of claims 1 to 3 for preparing a compound of formula I wherein $Z_1$, $Z_2$ and $R_1$ denote H, characterised in that a nitrosation reagent is reacted with the compound of formula V and the resulting compound is reduced by catalytic hydrogenation.

5. Process according to one of claims 1 to 3 for preparing a compound of formula I wherein $Z_1$, $Z_2$ and $R_1$ denote H, characterised in that a nitrosation reagent is reacted with the compound of formula V and the nitroso derivative obtained is reduced by means of a formamide.

6. Process according to one of claims 1 to 3 for preparing a compound of formula I wherein $Z_1$, $Z_2$ and $R_1$ represent H, characterised in that nitration of the compound of formula V is carried out, followed by reduction of the resulting compound.

7. Process according to one of claims 1 to 3, for preparing a compound of formula I wherein $Z_1$ and $Z_2$ represent H and $R_1$ denotes a $(C_{7-20})$alkyl group, characterised in that bromination of the compound of formula V is carried out and an amine of formula $H_2NR_1$ is reacted with the resulting compound.

8. Process according to one of claims 3 to 7 for preparing a compound of formula I wherein $Z_1$ and $Z_2$ together represent a carbonyl or thiocarbonyl group, characterised in that the compound of formula II is reacted with urea or thiourea, respectively.

9. Process according to one of claims 1, 3 and 7, for preparing a compound of formula I wherein $Z_1$ and $Z_2$ together represent a carbonyl or thiocarbonyl group and $R_2$, $R_3$, and $R_4$ represent H, characterised in that the urea or thiourea, respectively, is reacted with the compound of formula

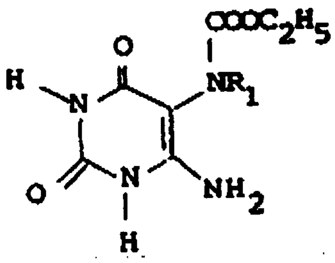

wherein $R_1$ denotes a $(C_{7-20})$alkyl group, obtained by debenzylation of

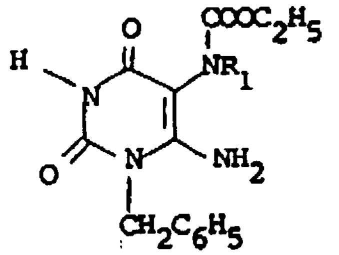

**Patentansprüche**

**Patentansprüche für folgende Vertragsstaaten : AT, BE, CH, DE, DK, FR, GB, GR, IT, LI, LU, NL, SE**

1. Verbindung der Formel

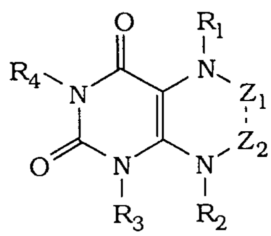

I

in der
$R_1$ H oder $(C_7-C_{20})$-Alkyl,
$R_2$ H oder $(C_7-C_{20})$-Alkyl,
$R_3$ H, $CH_3$ oder $(C_7-C_{20})$-Alkyl,
$R_4$ H, $CH_3$ oder $(C_7-C_{20})$-Alkyl,
$Z_1$ und $Z_2$ jeweils H oder gemeinsam eine Carbonyl- oder Thiocarbonylgruppe bedeuten,
mit der Maßgabe, daß ein einziger der Substituenten $R_1$ bis $R_4$ eine $(C_7-C_{20})$-Alkylgruppe darstellt und eine der Gruppen $R_3$ oder $R_4$ von H verschieden ist, wenn $R_1$ n-Dodecyl bedeutet,
und deren Additionssalze mit einer pharmazeutisch annehmbaren Säure.

2. Verbindung nach Anspruch 1, **dadurch gekennzeichnet**, daß eine der Gruppen $R_1$, $R_2$, $R_3$ oder $R_4$ eine $C_{12}$-$C_{20}$-Alkylgruppe darstellt.

3. Verbindung nach einem der Ansprüche 1 oder 2, **dadurch gekennzeichnet**, daß $Z_1$ und $Z_2$ jeweils H bedeuten.

4. Verbindung nach einem der Ansprüche 1 oder 2, **dadurch gekennzeichnet**, daß $Z_1$ und $Z_2$ gemeinsam eine Carbonyl- oder Thiocarbonylgruppe bedeuten.

5. Verbindung nach einem der Ansprüche 1 oder 2, **dadurch gekennzeichnet**, daß $Z_1$ und $Z_2$ gemeinsam die Carbonylgruppe bedeuten.

6. Arzneimittel, enthaltend als Wirkstoff eine Verbindung nach Anspruch 1.

7. Arzneimittel nach Anspruch 6, **dadurch gekennzeichnet**, daß $R_1$, $R_2$, $R_3$ oder $R_4$ eine $C_{12}$-$C_{20}$-Alkylgruppe bedeuten.

8. Arzneimittel nach einem der Ansprüche 6 oder 7, **dadurch gekennzeichnet**, daß $Z_1$ und $Z_2$ jeweils H bedeuten.

9. Arzneimittel nach einem der Ansprüche 6 oder 7, **dadurch gekennzeichnet**, daß $Z_1$ und $Z_2$ gemeinsam eine Carbonyl- oder Thiocarbonylgruppe bedeuten.

10. Verfahren zur Herstellung der Verbindungen nach einem der Ansprüche 1 bis 5, **dadurch gekennzeichnet**, daß man

1°) eine Aminogruppe der Formel $NR_1Z_1$ in die 5-Stellung einer Verbindung der Formel

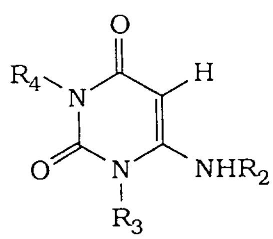

V

einführt,

- entweder durch Reduktion einer durch Nitrosierung oder Nitrierung in die 5-Stellung dieser Verbindung (V) eingeführten NO- oder $NO_2$-Gruppe zur Bildung der Verbindungen, worin $Z_1$ = $R_1$ = H bedeuten,
- oder durch Einwirkung von $HNR_1Z_1$ auf das durch Bromierung dieser Verbindung (V) erhaltene, in der 5-Stellung bromierte Derivat zur Bildung der Verbindungen, worin $Z_1$ = H und $R_1$ - ($C_7$-$C_{20}$)-Alkyl bedeuten,

  und gegebenenfalls zur Bildung der Verbindungen, worin $Z_1$ und $Z_2$ gemeinsam die Carbonyl- oder Thiocarbonylgruppe bedeuten,

2°) Harnstoff oder Thioharnstoff auf die Verbindung der Formel

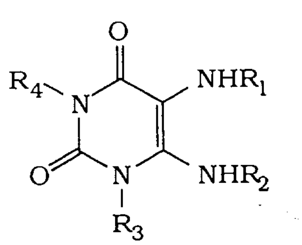

II

einwirken läßt, gegebenenfalls über das Carbamat in der 5-Stellung, wenn $R_1$ ($C_7$-$C_{20}$)-Alkyl und $R_2$ H bedeuten.

**Patentansprüche für folgenden Vertragsstaat : ES**

1. Verfahren zur Herstellung einer Verbindung

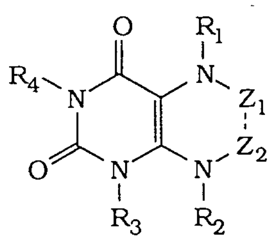

I

in der
$R_1$ H oder ($C_7$-$C_{20}$)-Alkyl,
$R_2$ H oder ($C_7$-$C_{20}$)-Alkyl,
$R_3$ H, $CH_3$ oder ($C_7$-$C_{20}$)-Alkyl,
$R_4$ H, $CH_3$ oder ($C_7$-$C_{20}$)-Alkyl,
$Z_1$ und $Z_2$ jeweils H oder gemeinsam eine Carbonyl- oder Thiocarbonylgruppe bedeuten,
mit der Maßgabe, daß ein einziger der Substituenten $R_1$ bis $R_4$ eine ($C_7$-$C_{20}$)-Alkylgruppe darstellt und eine der Gruppen $R_3$ oder $R_4$ von H verschieden ist, wenn $R_1$ n-Dodecyl bedeutet,
und von deren Additionssalzen mit einer pharmazeutisch annehmbaren Säure, **dadurch gekennzeichnet**, daß man

1°) eine Aminogruppe der Formel $NR_1Z_1$ in die 5-Stellung einer Verbindung der Formel

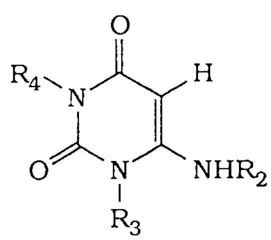

V

einführt,

- entweder durch Reduktion einer durch Nitrosierung oder Nitrierung in die 5-Stellung dieser Verbindung (V) eingeführten NO- oder $NO_2$-Gruppe zur Bildung der Verbindungen, worin $Z_1$ = $R_1$ = H bedeuten,
- oder durch Einwirkung von $HNR_1Z_1$ auf das durch Bromierung dieser Verbindung (V) erhaltene, in der 5-Stellung bromierte Derivat zur Bildung der Verbindungen, worin $Z_1$ = H und $R_1$ - ($C_7$-$C_{20}$)-Alkyl bedeuten,
  und gegebenenfalls zur Bildung der Verbindungen, worin $Z_1$ und $Z_2$ gemeinsam die Carbonyl- oder Thiocarbonylgruppe bedeuten,

2°) Harnstoff oder Thioharnstoff auf die Verbindung der Formel

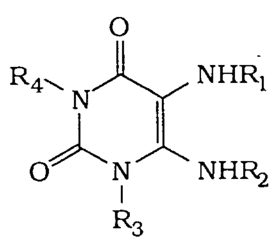

II

einwirken läßt, gegebenenfalls über das Carbamat in der 5-Stellung, wenn $R_1$ ($C_7$-$C_{20}$)-Alkyl und $R_2$ H bedeuten.

**2.** Verfahren nach Anspruch 2, **dadurch gekennzeichnet**, daß man die Verbindungen der Formel I, worin $Z_1$ und $Z_2$ H bedeuten, durch Einwirkung einer pharmazeutisch annehmbaren Säure in die Salze überführt.

**3.** Verfahren nach einem der Ansprüche 1 oder 2, **dadurch gekennzeichnet**, daß eine der Gruppen $R_1$, $R_2$, $R_3$ oder $R_4$ eine $C_{12}$-$C_{20}$-Alkylgruppe darstellt.

**4.** Verfahren nach einem der Ansprüche 1 bis 3 zur Herstellung einer Verbindung der Formel I, in der $Z_1$, $Z_2$ und $R_1$ H bedeuten, **dadurch gekennzeichnet**, daß man ein Nitrosierungsreagens auf die Verbindung der Formel V einwirken läßt und die erhaltene Verbindung durch katalytische Hydrierung reduziert.

**5.** Verfahren nach einem der Ansprüche 1 bis 3 zur Herstellung einer Verbindung der Formel I, in der $Z_1$, $Z_2$ und $R_1$ H bedeuten, **dadurch gekennzeichnet**, daß man ein Nitrosierungsreagens auf die Verbindung der Formel V einwirken läßt und das erhaltene Nitrosoderivat über ein Formamid reduziert.

**6.** Verfahren nach einem der Ansprüche 1 bis 3 zur Herstellung einer Verbindung der Formel I, in der $Z_1$, $Z_2$ und $R_1$ H bedeuten, **dadurch gekennzeichnet**, daß man eine Nitrierung der Verbindung der Formel V, gefolgt von einer Reduktion der erhaltenen Verbindung durchführt.

**7.** Verfahren nach einem der Ansprüche 1 bis 3 zur Herstellung einer Verbindung der Formel I, in der $Z_1$ und $Z_2$ H und $R_1$ eine ($C_7$-$C_{20}$)-Alkylgruppe bedeuten, **dadurch gekennzeichnet**, daß man eine Bromierung der Verbindung der Formel V bewirkt und die erhaltene Verbindung mit einem Amin der Formel $H_2NR_1$ umsetzt.

**8.** Verfahren nach einem der Ansprüche 3 bis 7 zur Herstellung einer Verbindung der Formel I, in der $Z_1$ und $Z_2$ gemeinsam eine Carbonyl- oder Thiocarbonylgruppe bedeuten, **dadurch gekennzeichnet**, daß man die Verbindung der Formel I mit Harnstoff bzw. Thioharnstoff umsetzt.

**9.** Verfahren nach einem der Ansprüche 1, 3 und 7 zur Herstellung einer Verbindung der Formel I, in der $Z_1$ und $Z_2$ gemeinsam eine Carbonyl- oder Thiocarbonylgruppe und $R_2$, $R_3$ und $R_4$ H bedeuten, **dadurch gekennzeichnet**, daß man die Verbindung der Formel

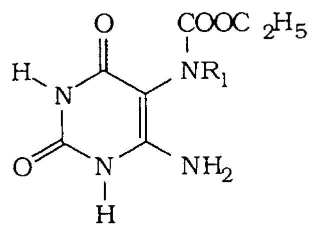

in der $R_1$ eine ($C_7$-$C_{20}$)-Alkylgruppe darstellt, die man durch Debenzylierung von

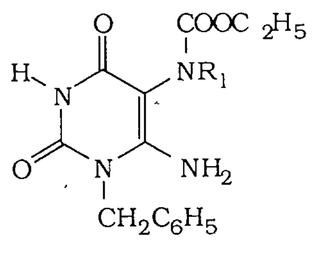

erhalten hat, mit Harnstoff bzw. Thioharnstoff umsetzt.